# EUROPEAN PATENT SPECIFICATION

(11) **EP 4 071 766 B1**
(45) Date of publication and mention of the grant of the patent: **19.03.2025**
(21) Application number: 22167174.6
(22) Date of filing: 07.04.2022
(51) Int. Cl.: G16H 40/63, G16H 40/67, G16H 50/80

(54) **MONITORING AND MANAGEMENT OF THE PSYCHO-PHYSICAL CONDITIONS OF A PATIENT**
ÜBERWACHUNG UND VERWALTUNG DES PSYCHO-PHYSISCHEN ZUSTANDS EINES PATIENTEN
SURVEILLANCE ET GESTION DES CONDITIONS PSYCHOPHYSIQUES D'UN PATIENT

(30) Priority: 08.04.2021 IT 202100008828
(43) Date of publication of application: 12.10.2022
(73) Proprietor: Salute 3D S.r.l., 85100 Potenza (IT)
(72) Inventor: STRAZIUSO, Nicola, 85100 Potenza (IT)
(74) Representative: Studio Torta S.p.A.

(56) References cited:
- WO-A1-2016/110804
- WO-A1-2021/050910
- WO-A2-2010/102069
- US-A1- 2018 325 407

## Description

### CROSS-REFERENCE TO RELATED APPLICATIONS

This Patent Application claims priority from Italian Patent Application No. 102021000008828 filed on April 8, 2021.

### FIELD OF THE ART

The present invention relates to the monitoring and management of the psycho-physical conditions of a subject definable as a user, healthy and potentially a patient, who over time or due to unforeseen and unpredictable events becomes a patient, and in particular to a platform and to a relative method for monitoring parameters relative to the psycho-physical conditions of a user/patient. In the following, for the sake of simplicity and in order to ensure a better understanding of the invention, the terms user and patient will be used alternately with each other to refer to the same entity, namely the person (female/male) who can become a patient.

In particular, and without any loss of generality, the invention finds mainly application in the social-health field, in particular to detect and monitor parameters relative to the psycho-physical condition of a user and to set up the station and the facilities for the administration of medical services if required by the psycho-physical conditions.

Furthermore, the present invention finds particular application during health emergencies, such as the Sars-CoV-2 pandemic, where the incidence and the influx of patients, or potential patients, is higher.

### BACKGROUND

As is known, health emergencies, such as the known and current pandemic due to the Sars-CoV-2 virus (commonly known as Covid-19), bring about a substantial change in people's daily lives; in particular, the Covid-19 pandemic has led to a substantial and necessary reduction in personal contacts in order to avoid an excessive and potentially uncontrolled spread of the virus.

It is also known that during a health emergency, in particular during the current Covid-19 pandemic, the incidence and influx of patients to medical facilities, such as hospitals or private facilities, increases almost exponentially; as a result, medical and hospital staff find themselves having to manage a large and often uncontrolled flow of patients, which, particularly during the Covid-19 pandemic, brought the health system in many countries to its knees.

In this particular historical period, the administration of medical services remotely (where possible) has become increasingly important, as it makes it possible to limit the contacts between doctor and patient and, therefore, to limit in a non-negligible way any a possible viral spread. By way of example, clinical diagnostics has undergone considerable developments, some of which have enabled medical staff to diagnose a patient remotely, thus combining medical expertise with IT skills. This new type of patient management and diagnosis is commonly known as telemedicine ("telehealth").

US 2018/325407 A1 discloses a CHF management system comprising a wearable textile-based harness and a signal acquisition unit.

WO 2010/102069 A2 discloses systems, devices, and methods with applications in particular in hospitals and other patient care facilities. In particular, the systems, devices, and methods can be used in particular for acquiring physiological information from patients, analyzing the physiological information, and communicating the physiological information to clinicians and other systems or devices.

WO 2016/110804 A1 discloses one or more wearable devices (i.e. attached or applied to limbs, body, head or other body extremities but also applicable to implanted or physiologically attachable systems) which have means of enabling diagnostic or prognostic monitoring applicable to monitoring relevant parameters and corresponding analysis determination and characterisation applicable to the onset or detection of events or health conditions of interest, in particular sleep monitoring and associate EEG sensors.

WO 2021 050910 A1 discloses open respirator devices having sensors for measuring breath biometrics.

### OBJECT AND SUMMARY OF THE INVENTION

Precisely because of the extraordinary period, the administration of remote medical services, such as telemedicine, can play a key role in the management and diagnosis of patients, whether they are suffering from known pathologies or from Covid-19 infection. Where such measures are not available or feasible for certain patients, in order to avoid overcrowding of medical facilities and possible further complications for already medically sensitive patients, as well as to correctly and efficiently administer to the patient the necessary medical services, the influx of patients must be duly regulated and efficiently managed.

The Applicant was able to observe that the technology currently available to support patients and medical staff in order to ensure the administration of medical services and, at the same time, to efficiently manage the same patients at medical facilities is susceptible to further improvements.

Firstly, the monitoring of the "baseline" parameters for the diagnosis of a patient (e.g. heartbeat, oxygen level, O₂, and the like) is currently only carried out at certain instants or lapses of time either automatically (e.g. with special measuring instruments, which are often not portable) or with the help of qualified medical staff, thus resulting in a limited amount of information relative to the patient's state of health; therefore, the doctor's ability to process a diagnosis, particularly when remotely, is constrained by the scarcity of available data on the patient's state of health.

In addition, the processing of the diagnosis also depends on the type of instrumentation used for the measurement; for example, such instrumentation may not be correctly calibrated and therefore produce unreliable and non-comparable results.

Another factor of considerable importance, particularly during the current global pandemic, is the psychological state of the patient, which can help in the management of the disease and subsequent treatment. The doctor is therefore also called upon to monitor the patient's psychological health and to accompany him on this front as well towards a prompt recovery. This need is most felt in the current pandemic period, where contact with reality and other people has been significantly reduced, leading to non-negligible repercussions on the psychological health of people and, even more so, of Covid-19 patients.

In addition, if a patient shows symptoms that require accurate monitoring and treatments in dedicated medical facilities, the patient will have to go to one of these medical facilities adapted to monitor his state of health and in order to receive the necessary treatments to return to a normal state of health. In order to better manage the patient, there is a need for close collaboration between electronic and IT systems for the remote management of the patient's state of health and the medical facilities, the latter being appropriately provided with electronic and IT tools to be able to communicate with the patient's treating physician and to set up the stations, medical staff and facilities that are necessary for the patient's stay. Currently, such efficient patient management is not always possible.

There is therefore a need to improve the current systems for managing the patient's state of health in order to facilitate the setting up of a network that allows the management of patients and medical resources (such as stations, staff and the like) from the diagnosis to the administration of treatments also in medical facilities. In particular, this need is even more felt in the management of Covid-19 patients.

Aim of the present invention is to address the need for improvement of the platforms and of the methods for monitoring and managing the psycho-physical conditions of a patient that emerges in the prior art.

According to the present invention, a platform and a relative method for monitoring parameters relative to the psycho-physical conditions of a patient, as defined in the appended claims, are realized.

### BRIEF DESCRIPTION OF THE DRAWINGS

To better understand the present invention preferred embodiments thereof will be now described, for exemplary and non-limiting purposes, with reference to the appended claims, wherein:
- Figure 1 schematically shows a platform for monitoring parameters relative to the psycho-physical conditions of a patient according to the present invention;
- Figure 2 shows a device for monitoring parameters relative to the psycho-physical conditions of a patient according to the present invention;
- Figure 3 schematically shows the time sequence of a method for monitoring parameters relative to the psycho-physical conditions of a patient according to the present invention; and
- Figure 4 schematically shows a method for monitoring parameters relative to the psycho-physical conditions of a patient according to the present invention.

### PREFERRED EMBODIMENTS OF THE INVENTION

The present invention will now be described in detail with reference to the accompanying figures to allow a skilled person to make and use it.

Unless otherwise defined, all the herein used technical and scientific terms have the same meaning commonly used by the ordinary skilled in the art of the present invention. In case of conflict, the present invention, including definitions provided, will be binding. Furthermore, the examples are provided for merely illustrative purposes and must not be regarded as limiting.

In particular, the block diagrams included in the enclosed figures and hereinafter described must not be considered as a representation of the structural features, i.e. construction limitations, but must be construed as a representation of functional features, namely inner properties of the devices and defined by the obtained effects i.e. functional limitations which can be implemented in different ways, so as to protect the functionality thereof (chance of functioning).

In order to ease the understanding of the herein described embodiments, reference will be made to some specific embodiments and a specific language will be used to describe them.

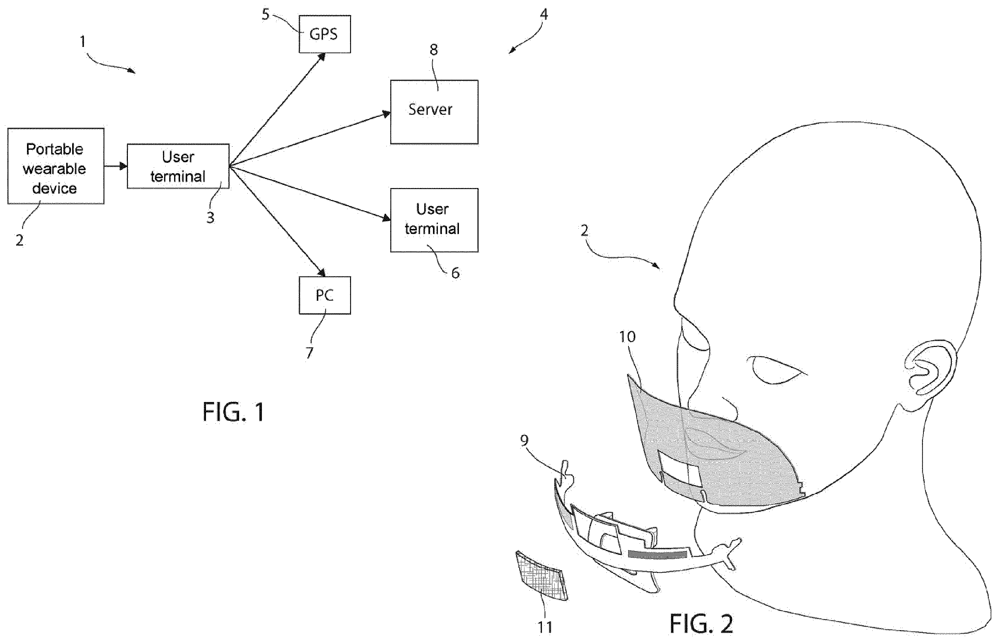

Figure 1 shows a hardware and/or software platform 1 configured to monitor parameters relative to the psycho-physical conditions of a patient; in the following, for simplicity of description and to allow a better understanding of the invention, unless otherwise indicated, reference is made to the monitoring of parameters relative to the psycho-physical conditions of a single patient, without this representing a limitation to the present invention; de facto, in further embodiments of the present invention, the platform 1 allows the monitoring of parameters relative to the psycho-physical conditions of more patients. In particular, the aforesaid patient (or the patients according to what is mentioned above) is an "alerted" patient and a candidate, in the presence of symptoms, for a more in-depth evaluation in an outpatient or inpatient medical facility.

The platform 1 comprises a portable wearable device 2, such as for example a mask like the one shown in Figure 2 and described in greater detail below, configured to monitor biometric and/or ambient parameters of a patient and adapted to generate and transmit data indicative of the detected biometric and/or ambient parameters. The platform 1 further comprises a user terminal 3 coupled to the portable wearable device 2 and configured to:
- receive the data relative to the biometric and/or ambient parameters of the patient detected by the portable wearable device 2;
- generate data relative to psychometric parameters relating to the psychological conditions; and
- process the data relative to the detected biometric and/or ambient parameters and to the psychometric parameters in order to generate an identifier containing information relative to the overall state of psycho-physical health of the patient and/or the surrounding environment.

The platform 1 further comprises external devices 4, coupled to the user terminal 3 and configured to receive and process the identifier.

The user terminal 3 is, for example, any user terminal, either fixed, such as personal desktop computers, or mobile type, such as personal laptop computers, tablets, phablets and smartphones, capable of connecting to the Internet network in a wired or wireless manner, for example via Wi-Fi, via a LAN or WLAN, or via the fixed or mobile telephone network, or any other current or future Internet network access technology. In the present embodiment, the user terminal 3 is a mobile device, such as, for example, a smartphone belonging to the patient.

In the present embodiment, the user terminal 3 is coupled to the portable wearable device 2 via a wireless connection (e.g., via Bluetooth connection); similarly, the external devices 4 and the user terminal 3 are coupled to each other wirelessly. In further embodiments, the coupling between the user terminal and the portable wearable device 2 may take place, for example, through further wireless connection technologies, such as, for example, wireless Internet connection and the like.

The user terminal 3 is also coupled, by means of a related geolocalisation module (not shown), to localization devices 5 configured to locate the geographical position of the patient's user terminal 3. By way of example, the localization devices 5 are GPS (*Global Positioning System*) devices, e.g. using the Galileo positioning protocol. The localization devices 5 are also configured to generate routes for taking the patient to the medical facility if a first state of health, hereinafter referred to as "not healthy", is detected; these routes are made available to the patient by means of the user terminal 3, for example through a navigation software (app). It should be noted that, by "not healthy" state of health, as also better explained below, it is meant a state of health in which one or more of the biometric and/or ambient parameters and the psychometric parameters exceed predetermined threshold values indicative of normal health conditions. By way of example, a "not healthy" state of health (i.e., the first state of health) may be identified as a state of health in which the patient's blood oxygen level, the heartbeat, and the degree of anxiety are above respective average normal threshold values and are also considered by the treating physician to be indicative of an abnormal state of health requiring the administration of *ad hoc* medical services.

In the present embodiment, the identifier is, for example, a dynamic QR code, such that when the biometric and/or ambient parameters and/or the psychometric parameters change, this identifier is updated accordingly. In other words, the identifier is indicative of the patient's state of health and is designed, if it is indicative of a "not healthy" state of health, to:
- ensure that the treating physician is notified of an abnormal health condition of the patient;
- ensure that the patient is taken to a medical facility, in particular thanks to the geolocalisation devices 5;
- if the medical facility is able to receive the patient, to ensure that the medical facility is notified of the patient's state of health and is organized to administer the medical services necessary for the patient's care;
- update and/or add the state of health to "healthy", for example following the healing of the patient, in order to allow access to organizations, e.g. public and private (e.g. work, leisure and similar places).

The user terminal 3 is also provided with a software, here an app called Sani, such that, when run, it causes the user terminal 3 to perform the above operations for which it is configured.

The external devices 4 comprise:
- user terminals 6 configured to send requests for sharing of the identifier generated by the user terminal 3 and receive said identifier upon acceptance of the sharing by the user terminal 3 as instructed by the patient;
- hardware and/or software IT resources 7 (such as, for example, workstations and notebooks or the like), one is shown in Figure 1, configured to receive and/or store the identifier from the user terminal 3, possibly following a request for sharing; and
- servers 8, one is shown in Figure 1, configured to receive the identifier from the user terminal 3 and/or data relative to the medical facility and to the state of health of the patient during administration of the medical services, possibly following a request for sharing.

The user terminals 6 are, for example, any user terminals, either fixed, such as personal desktop computers, or mobile type, such as personal laptop computers, tablets, phablets and smartphones, capable of connecting to the Internet network in a wired or wireless manner, for example via Wi-Fi, via a LAN or WLAN, or via the fixed or mobile telephone network, or any other current or future Internet network access technology. In the present embodiment, the user terminals 6 are mobile devices, such as, for example, smartphones or tablets held by medical staff of a medical facility or by trusted persons, for example drivers or relatives of the user, as well as other users or organizations.

The IT resources 7 and/or the user terminals 6 are for example electronic and IT systems of organizations (public and/or private, commercial and the like) and of medical facilities, such as private facilities or hospitals, and are further configured to initiate a patient management process if the identifier received from the user terminal 3 is indicative of a need for the patient to stay in the medical facility.

The server 8 is for example a remote server, connected to one or more medical facilities and designed to store data relative to the medical facility and/or the influx of patients into it.

In the case of the user terminal 6, of the IT resource 7 or of the server 8, the user terminal 3, by means of the Sani app, is configured to transmit the identifier following a possible request for sharing to one of the aforesaid external devices 4. In particular, in the case of the IT resource 7 or of the server 8, the user terminal 3 can, by sending the identifier (i.e., by communicating the patient's state of health), initiate the procedure for managing the stay of a patient in the medical facility, as better described in the following paragraphs.

With reference to Figure 2, the portable wearable device 2 is a wearable type device; in the present embodiment, the portable wearable device 2 is a smart mask that the patient wears periodically (i.e., for predefined periods of time, possibly prescribed by the treating physician) or continuously (i.e., for the entire day). The portable wearable device 2 comprises:
- a supporting structure 9, configured to carry a plurality of sensors configured to detect the biometric and/or ambient parameters; and
- a protective shield 10, carried by the supporting structure 9.

In particular, the supporting structure 9 is designed to provide spaces in contact with the soma of the user wearing the portable wearable device 2.

By way of non-limiting example, the plurality of sensors comprises: micro- and/or nanobiosensors, ambient micro- and/or nanosensors, thermal sensors, biometric readers with infrared detectors, sensors for detecting carbon dioxide (CO₂) and sensors for detecting fine particulate (e.g. using an infrared (IR) measurement method).

As anticipated above, the portable wearable device 2, in particular the plurality of sensors, is designed to detect biometric and/or ambient parameters, wherein:
- biometric parameters are indicative of the state of health of the patient and comprise at least one of body temperature, oxygen (O₂) saturation, CO₂ concentration, cardiac frequency, voice analysis, analysis of cough as well as breadth intensity and quality; and
- ambient parameters are indicative of the ambient conditions surrounding the patient and comprise at least one of quantity of CO₂ and fine particulate in the air.

The protective shield 10 has a multi-layer structure, comprising a plurality of layers (not shown). The protective shield 10 is designed to:
- have optical properties, such as for example antireflection, anti-scratch and anti-fog properties;
- be water-repellent and self-cleaning;
- allow protection from droplets of saliva;
- have a longer exposure time;
- be easy to clean and disinfect;
- be capable of not interfering with the patient's breathing, phonation and swallowing patterns; and
- avoid the possibility of respiratory acidosis and alteration of the cutaneous, oral, nasal, pulmonary microbiota and, more generally, of the respiratory tracts, which are contemplated when using surgical or closed masks.

The supporting structure 9 is configured to carry further electronic components, such as a digital microphone (e.g., a microphone wireframe), a digital acoustic receiver, one or more Lab-On-Chips (LOCs) with relative circuits for reconfiguring the same LOCs, Internet-of-Things (IoT) systems, embedded circuit systems enabled for the comparison with matrix and experimental data stored in a remote database, (such as, for example, the server 8) and systems for transmitting data acquired by the plurality of sensors; in particular, the latter systems for transmitting the acquired data comprise, for example, wireless communication modules (e.g. a Bluetooth beacon for communication with the Sani app on the user terminal 3). The additional electronic components carried by the supporting structure 9 are also integrated as wireframes between the layers of the protective shield 10. Note that the plurality of sensors and the additional electronic components are suitably connected to each other by a plurality of conductive tracks of metallic material, not shown. In addition, according to one aspect of the present invention, the plurality of conductive tracks is configured to define a layer with photovoltaic properties.

The portable wearable device 2 further comprises a filter 11 carried by the supporting structure 9 and by the protective shield 10 and arranged in a central position with respect to the latter; in particular, the filter 11 is configured to detect in further detail some aspects if required by the state of health and/or the ambient conditions. According to one embodiment of the present invention, the aforesaid aspects can, if necessary, be defined in greater detail through, for example, the insertion of nano-structured sensors in the portable wearable device 2 and/or in the filter 11; the nanosensors are designed to sample the air flow (inflow and outflow) passing through the respiratory orifices (i.e., nose and mouth) and to evaluate the quality of the air exhaled and inhaled by the user (measurable through, for example, a flowmeter). In addition, the aforesaid nanosensors, according to the above sampling and evaluation, are alternatively or sequentially configured to:
- activate a wireframe, integrated in the portable wearable device 2 and/or in the same filter 11, configured to produce and/or deliver therapeutic ozone; and
- allow the insertion of specific swabs for carrying out rapid anti-viral and/or antibacterial tests in the same filter 11 and/or in the portable wearable device (2).

Furthermore, the filter 11 is configured to carry out a feedback and/or feed-forward correction, also referred to as rectification, of the air quality modifying the microclimate, in particular by heating the air with a special wireframe (also integrated in the portable wearable device 2) or by enriching it with oxygen by means of a further wireframe configured to generate ozone; in this way, the portable wearable device 2 allows for the creation of a climate or a microclimate the conditions of which are favorable to the patient's health and unfavorable to the survival, residence and proliferation of viruses. In one embodiment of the invention, the filter 11 is further designed to provide a "nano-architecture" specular to the shape, dimensions and spatial representation of a particular virus, thus being able to "trap" it. In another embodiment, the filter 11 is of a removable type, namely it can be removed, for example, to be analysed in structural or microbiological terms in order to find a known virus and/or a variation thereof, and replaced.

According to one aspect of the present invention, the filter 11 has a "nano-architecture", in particular having a single-cuvette or multiple-cuvette shape, the walls and the interfaces thereof with the soma of the user (e.g., in contact with the chin muscle of the same user) are coated with electromagnetic wireframes allowing the release, through micro- or nano-electroporation, of vaccine forms (such as, for example, attenuated viruses, capsids or viral proteins) suitably prepared in specialised laboratories. The supply, which can also take place at home, of the vaccine forms by the filter 11 takes place once the filter 11 has been inserted in the supporting structure 9 and the connection to the supporting structure 9 as well as to any external control systems has been verified; in greater detail, the filter 11, once inserted and connected, remains in stand-by for a period of time such as to synchronize the simultaneous vaccination (i.e., the supply of the vaccine form by the filter 11) of a large number of people, for example millions, and to facilitate the maximum response and effectiveness of the vaccination campaign.

According to one embodiment of the present invention, the portable wearable device 2 is manufactured using the machinery described in Italian patent application No. 102017000034142.

In general, the portable wearable device 2 is manufactured using direct digital manufacturing and processing techniques and processes, such as for example additive manufacturing, competitive sustainable manufacturing, that is repeatable and competitive, and biotechnology manufacturing with advanced materials, such as advanced nanometric materials (biotechnology and advanced materials). The aforesaid manufacturing techniques and processes allow the finalisation and/or the realization of objects, such as the portable wearable device 2, by layering and the selective, controlled and three-dimensional deposition of materials with deposition techniques, such as, for example, pulsed electron deposition on three-dimensional, 3D, movement, with up to fourteen degrees of freedom with nanometric precision of different nature and composition.

The portable wearable device 2 is thus obtained by layering different materials, comprising for example:
- biocompatible materials, such as quaternary systems of silicon oxide, calcium oxide, phosphoric anhydride and sodium oxide (SiO₂-CaO-P₂O₅-Na₂O);
- materials for buffer layers, such as aluminium, titanium or cerium oxides, strontium titanate or barium fluoride (AlOₓ, TiOₓ, CeOₓ, SrTiO₃ and BaF₂);
- colossal magnetoresistance (CMR) manganites, i.e. materials with a Curie temperature of 350 K and 100% spin polarisation at room temperature;
- hard covering materials such as silicon carbide and titanium nitride (SiC and TiN);
- organic materials, such as teflon and polypropylene;
- transparent conductive oxides (TCO), such as ITO, IMO and zinc oxide (ZnO);
- coloured pigments, such as zinc sulphide and potassium dichromate (ZnS and Cr₂Ot.xH₂O);4
- high band gap semiconductors, such as silicon oxides and a sulphur, germanium and copper alloy (SiOx and CuGS); and
- high-temperature superconducting materials (*High T_{c} Superconductors*)*,* i.e. having a critical temperature above 92 K and a critical current between 2 and 4 MA/cm², such as YBCO.

The plurality of sensors and the additional electronic devices of the portable wearable device 2 are thus made using advanced techniques that enable their high miniaturization; in other words, according to one aspect of the present invention, it is possible to obtain sensors and components of micro- and/or nanometric dimensions so as to obtain a compact and portable wearable device 2 that can be carried at any time.

The portable wearable device 2, once the biometric and/or ambient parameters have been acquired, is configured to transmit the latter as data to the user terminal 3, in particular to the Sani app, which receives and processes them so as to generate the identifier; in particular, the identifier is indicative of a clinical pre-certification of the patient, i.e. the data collected are an index for defining the diagnosis of the patient. In terms of the priority scale of the data received for processing the identifier, the user terminal 3, in particular the Sani app, receives in sequence:
- living context data, comprising data on the patient's date of birth, gender and living context;
- biometric indices, comprising data on normality parameters; and
- stress test data, comprising data on the degree of adaptability.

The stress test data represent the psychometric parameters relating to the psychological conditions of the patient and are determined according to the results obtained by the patient by periodically carrying out psychological tests, which are provided to the patient and/or chosen and approved by the patient via the Sani app. The psychological tests provided propose sets of questions to the patient, the answers to which are acquired and processed by the user terminal 3 to generate data indicative of the psychological state of the patient. By way of example, the psychological tests are structured to check the consistency of the answers given by the patient to repetitive and/or directly/indirectly formulated questions in order to test whether the inconsistent answer depended, for example, on a disturbance of vigilance and/or attention and which was previously better and which in the particular circumstance has worsened. Therefore, together with the data indicative of the biometric and/or ambient parameters detected by the portable wearable device 2, the data indicative of the psychometric parameters relating to the psychological conditions of the patient, detected by means of psychological tests, contribute to defining a clinical picture of the patient; in other words, the user terminal 3 is able to receive data relating to the psycho-physical state and verify that the values fall within the medical definition of healthy. By way of example, and particularly in the context of the current global pandemic, the patient may not be ill but may experience a progressive worsening of his psychological conditions, for example due to the discomfort in wearing a mask almost constantly, in dealing with the illness of a relative or friend, due to the impossibility of being able to carry out normal social activities and the like.

It should be noted that the patient is enabled to use the Sani app only following a registration procedure, which involves entering the patient's personal data (first name, surname, date of birth) and access credentials (e-mail and password). The sign-up is subject to a verification by e-mail. It should also be noted that, once the patient has signed up, he can view information pages on the Sani app as well as, for example, on the psychological tests that the patient can take.

The user terminal 3, in particular the Sani app, is designed to communicate with one or more of the external devices 4 to exchange some or all of the information detected by the portable wearable device 2 and processed by the user terminal 3. In particular, the patient, through the graphical interface of the Sani app, is able to select which of the external devices 4 available can receive the identifier or portions of the information contained therein (such as a portion of the biometric and/or ambient parameters, e.g. the patient's temperature or the use or frequency with which the portable wearable device 2 is worn by the patient).

In addition, the Sani app can be synchronised with other apps on the market that can be installed on the user terminal 3, such as, for example, the Immuni app or apps that are adapted to measure the biometric and/or ambient parameters of the patient wearing the portable wearable device 2; in other words, it is possible to establish a synchronised cooperation between the Sani app and the other apps made available to the patient. For example, if a patient comes into contact with a person who tested or has tested positive for Covid-19 and is notified via the Immuni app, the Sani app can monitor the degree of stress and anxiety upon receipt (thus acquire the psychometric parameters indicative of the patient's psychological conditions), as well as subsequent to this, of the contact notification; the Sani app is also designed to schedule a visit to a medical facility in the presence of any symptoms (identified by means of the data received by the portable wearable device 2), as better described below.

In general, the Sani app installed on the user terminal 3 is designed to be managed by three subjects:
- the user using the user terminal 3;
- an organization; and
- an administrator.

The user, here the patient, represents the "end user", i.e. the subject who is able to carry out the psychometric tests provided by the Sani app, to view the results of the aforesaid tests, to receive information about the state of health (monitored by the portable wearable device 2 and by the user terminal 3, specifically by monitoring biometric and/or ambient parameters by the portable wearable device 2 and psychometric parameters by the user terminal 3) and to scan the identifiers of further user terminals (e.g. the user terminals 6, belonging to further users) to verify their state of health, for example expressed as a percentage on a specific screen.

The organization represents a medical facility, whether public or private, or additional facilities and is able to scan the identifier associated with the user of the user terminal 3 by establishing filters (referred to below as scanning policies) that allow the results of the test carried out on the user, i.e. on the patient, to be displayed.

The administrator, also known as *admin,* represents the subject who manages the Sani app and its operation, for example by creating new questionnaires to check the psychological state of the user terminal 3; in particular, in this case, the administrator has full freedom over the structure of the questionnaires, as he is able to create questions , associate choices of different types to the same questions and associate questions with consistency in order to verify the psychological state of the user (i.e., to acquire the psychometric parameters indicative of the psychological conditions of the patient); in addition, the administrator is able to exclude that the answers given by the patient to the questionnaires proposed by the Sani app are not appropriate to the context and that they are not random. In addition, the answers given to the proposed questions can be associated with a range of results relating to the psychological state of the user. The administrator may also manage the users of respective user terminals 3, 6, manage information pages or the like to be proposed to the user via the Sani app, to view the carried out tests and to send notifications, e.g. push and/or email, to the user via the Sani app, e.g. to alert the user to carry out a psychological test or that the test results are available. By way of example, the graphical user interface of the Sani app can have a dashboard in which both the notifications and, for example, the information pages and/or the carried out tests that can be provided by the administrator can be displayed.

In this way, the psychological aspects, in conjunction with the aspects relating to the patient's physical health, are systematically controlled by the user terminal 3, as well as by the medical facility, in association with the portable wearable device 2.

Patients can also receive, via the Sani app installed on the user terminal 3, alternatively notifications relative to:
- failure to exceed the value of the biometric and/or ambient parameters measured with respect to the respective threshold values, i.e. confidence limits, indicative of a second state of health, referred to as "healthy"; or
- presence in a zone at risk, i.e. a zone where, for example, there are several people and there is a risk of gathering and non-adherence to social distancing.

It should be noted that a "healthy" state of health means a state of health in which one or more of the biometric and/or ambient parameters and the psychometric parameters are below the respective thresholds of predetermined values or within ranges of normal values and indicative of normal health conditions, namely opposite with respect to the "not healthy" state of health. By way of example, a "healthy" state of health can be identified as a state of health in which the patient's blood oxygen level, the heartbeat and the degree of anxiety are within the respective threshold values or within the normal ranges and are considered by the medical facility, and possibly the treating physician, to be indicative of a normal state of health and therefore not requiring the administration of *ad hoc* medical services.

It should also be noted that, if the value of the detected biometric and/or ambient parameters is exceeded with respect to respective threshold values, i.e. confidence limits, indicative of a second state of health, the user terminal 3 generates the notification following the processing of the biometric and/or ambient parameters detected by the portable wearable device 2 and the comparison with the confidence limits; in particular, the confidence limits are, for example, predefined by the administrator in a pre-structured, multi-parametric as well as comparative IT environment which processes in real time the data received from a number of user terminals 3 (in particular, the data relating to the biometric and/or ambient parameters), distributed over different territorial areas, for example municipal, provincial, regional, national and international ones and detectable by means of appropriate localization systems, such as, for example, the localization devices 5 or similar localization tools, for example of the GPS type. The data, which are indicative of distinctive and unique traits, are parameterised and crystallised in order to provide the basic information, as well as further information such as the reference context by zone, activity, mobility, age and gender. The variation of "a state of health" is detected and monitored if several states of health, associated with respective user terminals 3 and distributed in one or more territorial areas, vary in a period of time compatible with the evolution of a pathology; this variation of various states of health is therefore a potential candidate for comparison with known evolutionary profiles, characterized by peculiar transmissibility indices, in order to presage a possible endemic, epidemic or pandemic when the respective critical thresholds are exceeded. In this way, the territorial areas in which the variation of various states of health has been observed and in which one or more critical thresholds have been exceeded are classified as "at risk"; consequently, the administrator sends notifications to the involved players (i.e., the user devices 3, 6 and the external devices 4), one of which is for example the authorisation by the healthy user to:
- be tracked anonymously (i.e., by anonymously monitoring the localization data in a manner dissociated from the biometric parameters associated with the user of the user device 3) and only numerically; and
- activate the transfer of his own identifier (e.g. the QR code, indicative of his "healthy" state of health, i.e. the first state of health, dissociated from the user's personal data, so as to perform a *contact tracing*) in the event of a travel through this territorial area in order to reassure the people he meets, e.g. by notifying the user and/or the people who receive the identifier with a sound notification, for example personalized for the Sani app or for the user and/or the people who can receive the identifier. In further embodiments, the notification may be textual or a pop-up.

If, on the other hand, there is a zone at risk, classified by the administrator or by other recognised systems (national health system), i.e. a zone where, for example, there are several people and there is a risk of gathering and non-adherence to social distancing, the user terminal 3 is geolocalised with the help of the localization devices 5 by the administrator, who:
- compares the overlapping localization data within the maximum definition limits of the platform 1;
- identifies and follows the movement direction or "movement vector";
- determines the flow direction; and
- generates a notification, e.g. a sound one, such as a voice message from a library of sounds or messages, even personalized, aimed at maintaining optimal distancing.

At the request of the user of user terminal 3, the administrator can summarise the number of times the user has been on a collision course with other users and the zones involved.

In addition, the organization may receive:
- notifications of exceedances of critical thresholds in relation to users in order to take progressive and effective measures to contain pathologies;
- feedback and feed-forward control notifications; and
- real-time monitoring notifications of the spread of any pathologies.

In this way, the organization is able, as better described below, to set up the physical and human resources (i.e. beds, medical equipment and medical staff) for the management of the users for whom the Sani app has reported results requiring a monitoring in qualified facilities.

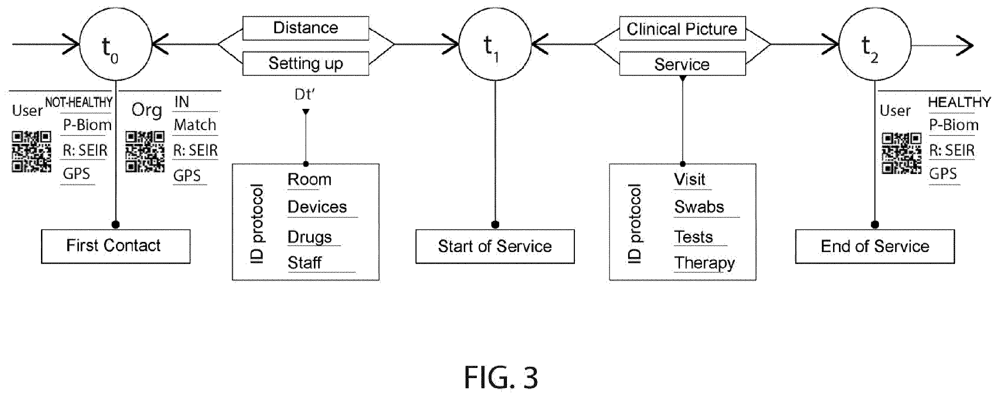

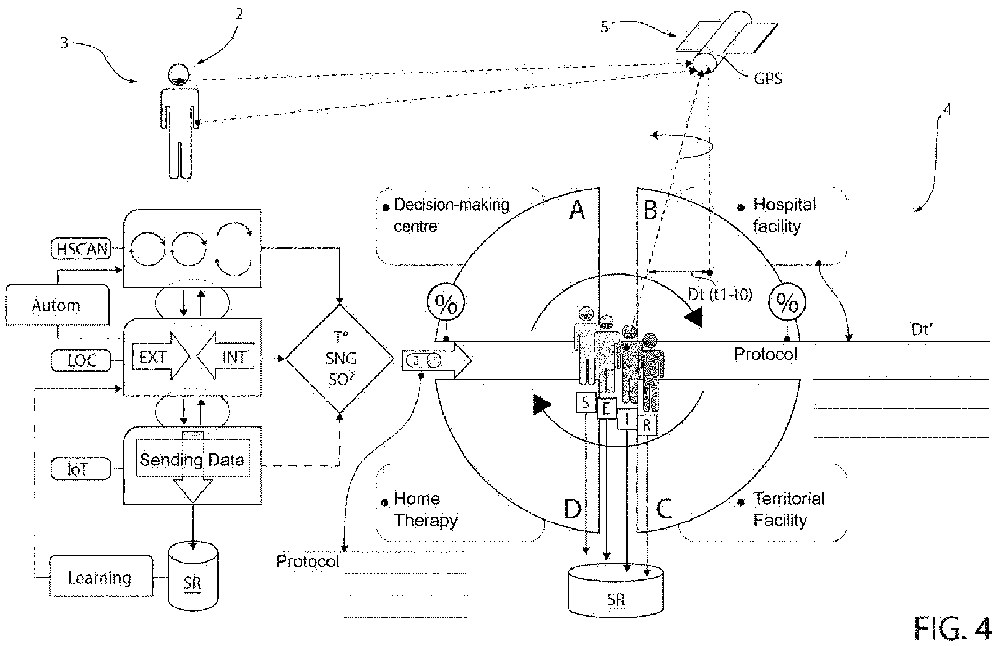

A method for monitoring parameters relative to the psycho-physical condition of a patient by a health system, such as a hospital or private facility, is now described with reference to Figure 3 and Figure 4. In particular, in the following, for simplicity of description, reference is made to a single patient, a single portable wearable device 2 and a single user terminal 3, provided with the Sani app. In addition and without any loss of generality, reference is made below to a method for monitoring parameters relative to the psycho-physical conditions of a patient in which the user terminal 3 and the portable wearable device 2 interface with the SEIR model, i.e. the management of the patient and his eventual transfer to a specialised medical facility takes place on the basis of classification parameters provided by the SEIR model. Note that the user terminal 3 and the portable wearable device 2 can interface with additional statistical probabilistic models of disease spread, here the Covid-19 virus.

In more detail, the SEIR model is a quantum model for predicting and monitoring and managing the spread thereof of the pandemic, starting from the classification of four types of subjects, identified by the following indications:
- [S], i.e. susceptible subjects (e.g. the elderly and chronically ill);
- [E], i.e. exposed persons (e.g. travellers, workers, students, health personnel);
- [I], or infected subjects; and
- [R], i.e. inpatients

The platform 1 according to the present invention is designed to interface with the SEIR model to share the starting terminology and to highlight the problems to be solved, i.e. the platform 1 according to the present invention is designed to:
- reduce the number of susceptible patients;
- strengthen the immune defences of exposed patients, such as travellers, for example students, workers and professionals, by reducing neuro-adaptive stress;
- reduce the duration and severity of the symptoms of the pathology, which leads to avoiding or reducing the incidence of hospitalization of potentially or actually infected patients; and
- avoid or reduce the number of severe cases requiring oxygen therapy and intensive care hospitalization.

In other words, the SEIR model dictates the guidelines for the operation of platform 1 and allows patients to be classified primarily on the basis of their health conditions (e.g. infected patients) and according to additional factors, such as, for example, the rate of exposure to risk situations, the transmission index (in particular, R0 and Rt) that recommend containment policies (e.g., social distancing, personal hygiene, use of personal protective equipment, isolation or lockdown) to limit the spread of the infection, promote the healing of infected persons, reduce the burden on medical facilities in order to avoid health system collapsing and facilitate the establishment or achievement of herd immunity.

The platform 1, also referred to as Service Delivery Platform, is therefore designed to perform the method according to the present invention and thereby enable the management of the response of the National medical service (NHS) and/or any private medical services in particular during the present Covid-19 pandemic. The method according to the present invention is therefore performed by means of the platform 1, in particular by exploiting the Sani app installed on the user terminal 3 and a program installable on one of the external devices 4 in use in one or more medical facilities in charge of patient management. According to one embodiment, this method provides for further periodic simulation phases in order to train operators and measure the reactivity of a medical facility (in particular, from the point of view of the availability of physical resources, such as healthcare equipment, and human resources, i.e. medical staff, the ability to reconfigure according to the flow of incoming and outgoing patients and the possibility of reaching the medical facility by the patient), in particular by checking the reactivity of the specialized wards to the treatment of patients with pathologies and, therefore, verifying the possibility of synchronising the healthcare services provided in the wards.

In addition, the platform 2 is designed to lend itself to epidemiological surveys, to update the pandemic plan of the different national health systems in synchronism and in concert with the guidelines of the World Health Organization (WHO), that is for awareness-raising, study and research campaigns.

According to one aspect of the present invention, therefore, the platform 2 constitutes a stable and reliable technological infrastructure capable of facilitating vaccination campaigns, of displaying in real time supply and demand data, in particular for the benefit also of the distribution and administration operations of the vaccines, of controlling the progress of the vaccination process in real time and refining it in a data-driven manner, i.e. according to the flow of data.

With particular reference to Figure 3, the platform 1 is designed to allow the management of the response of the NHS and/or private medical facilities according to the degree of impairment of each individual from the time instant in which the first symptoms are detected (referred to in the following as the first time instant t₀) and the time instant in which a patient arrives at the chosen medical facility (referred to in the following as the time instant t₁).

There follows now a description of an exemplary embodiment of the method according to one embodiment of the present invention. For simplicity of description, it is assumed that the questionnaire for the evaluation of the patient's psychological state has already been previously delivered and completed, i.e. that the psychometric parameters indicative of the patient's psychological conditions have already been collected.

In addition, it should be noted that, according to one aspect of the present invention, the Sani app installed on the user terminal 3 or the server 8 are designed to administer psychometric tests, even short ones, by audio-phonic means and elicit a response, for example of the affirmative/negative or multiple choice type (e.g., indicating a degree of adherence to the situation, for example of the type little, quite, very) and be decoded by algorithms, e.g., voice typing and speech recognition algorithms, to have the same result as that obtained using a keyboard; this aspect of the present invention may be implemented at any time, in particular, if it is deemed necessary, for example upon receipt of important data on the user's health.

In particular, in the following, it is assumed that the psychometric parameters are used by the platform in order to:
- evaluate the degree of prostration of the person;
- send comfort messages to promote recovery;
- facilitate communication with health personnel; and
- provide, after hospitalization, when the organic situation has stabilised and the psychometric parameters are indicative of a positive psychological evaluation even outside the hospitalization environment. If the psychometric parameters are indicative of a negative evaluation, the platform 1, in particular the user terminal 3, is designed to administer further tests via the Sani app and in a protected environment, i.e. under the observation of a health care professional; if there are also "psychiatric" traits, the user terminal 3 is designed to allow a request for a specialist visit.

In other words, it is assumed in the following that the hospitalization of a patient occurs under conditions mostly determined by the biometric parameters acquired by the portable wearable device 2, in particular when contingent and severe situations are detected, i.e. the patient is seriously ill.

It is also assumed that the reception, processing and generation of the identifier occur at the same first time instant t₀, i.e. without any delays due to the transmission and processing of the information.

At the first time instant t₀, the plurality of sensors of the portable wearable device 2 detects the biometric and/or ambient parameters and transmits them to the further electronic components, so that the latter convert them into data for the transmission to the user terminal 3. The user terminal 3 then receives the data transmitted by the portable wearable device 2 to process them and generate the identifier.

During processing, the user terminal 3 is designed to determine whether the data received from the portable wearable device 2 are data indicative of the first state of health ("not healthy"), i.e. one or more data have values beyond respective threshold values considered as indicative of a state of health that requires specific medical treatments. In other words, when symptoms of a pathology are detected, the user terminal 3 determines that an activation level has been reached.

It should be noted that the identifier is also generated according to the user's psychological state, evaluated according to the psychometric parameters derived from the patient's answers to the questionnaires provided by the Sani app.

It should also be noted that, in evaluating the state of health of the patient wearing the portable wearable device 2, the user terminal 3 may, according to one aspect of the present invention, associate a classification according to the SEIR model with the patient based on data received from the portable wearable device 2. In particular, each classification category of the SEIR model is characterized by respective threshold values which, if exceeded, determine a different classification of the patient; in particular, the threshold values are determined according to the biometric and/or ambient parameters of the patient, measured and received in the form of data by the portable wearable device 2, and to the geographical position of the patient, determined by means of the geolocalisation module of the user terminal 3 in association with the geolocalisation devices 5.

Once the first state of health ("not healthy") has been determined, the external devices 4 are designed to:
- send at least to the user terminal 3 a first notification indicating the need to provide medical assistance and/or to take the patient to a medical facility;
- send, to the medical facility, a second notification of the state of health of the patient to signal the need for medical assistance and/or hospitalization; and
- determine and store a first time Dt between the sending of the first notification and the beginning of the procedures for medical assistance and/or hospitalization by the medical facility and a second time Dt' between the sending of the second notification and the time for activation of the resources necessary for the medical assistance and/or hospitalization of the patient.

Note that medical assistance means any type of medical assistance available, e.g. remotely, at home or similar.

In particular, here, the Sani app is designed to simultaneously send notifications to the interested subjects, here the patient and the organizations (i.e. medical facilities in the territorial area of interest) that are the closest and that have configured their intervention protocols according to the fields present in the graphic interface of the software provided to them and installed on one of the external devices 4; in particular, in the fields of this software, one or more responsible doctors are envisaged, including the general practitioner if it is a National Health System (NHS) or a responsible doctor if it is a private facility. These data are managed by the administrator and are shared with the Sani app by the administrator.

In more detail, once the first ("not healthy") state of health of the patient has been determined, the organization is notified by means of, for example, a push notification and/or email; in particular, the Sani app can transmit the identifier to the organization immediately or later than the transmission of the notification of the first ("not healthy") state of health; in this case, the identifier represents a tool for defining the patient's clinical picture in further detail. According to one aspect of the present invention, during the determination of the first ("not healthy") state of health, the user terminal 3 is designed to assign a first class relating to the first ("not healthy") state of health of the patient according to a model, for example the SEIR model, at the first time instant t₀, i.e. it associates the respective classification according to the SEIR model, which is communicated to the organization jointly with the notification of the first ("not healthy") state of health with the biometric parameters and clinical symptomatology worthy of attention. It should be noted that the organization is, as anticipated, a medical facility, such as a hospital, chosen from among several medical facilities which, according to one aspect of the present invention, is chosen by the treating physician; this choice is made only after a delay beyond a pre-set time span according to certain protocols shared between the administrator and the organization (and, therefore, defined by both the administrator and the organization), in which the health conditions of the user/patient, monitored through the Sani app, are indicative of a worsening trend. According to one aspect of the present invention, beyond the aforesaid time span, pre-set by the administrator and the organization, and assuming that the treating physician has not chosen the medical facility by sending the request, the user, notified by the Sani app, is enabled to send the request to a facility selected from a list of suitable facilities and in his proximity, i.e. in a geographic position close to the user's geographic position. After a certain pre-set time span has elapsed without the user having sent the request, at a later stage, in which a "not healthy" state of health continues to be observed, the administrator automatically sends the request, so that the user can be accepted and treated by the selected medical facility. In particular, the observation of a "not healthy" state of health is observed by analysing the worsening trend of the biometric parameters; in particular, the biometric parameters are actively monitored through the portable wearable device 2 and their worsening trend is evolutionarily predicted by the platform 1 by comparing data relating to biometric parameters with data relating to biometric parameters predicted by the server 8, the latter being data relating to similar cases (also seasonal) stored in the server 8 and used as a comparison yardstick for the data relating to the biometric parameters of the patient. In other words, the platform 1 makes it possible both to check the trend of the biometric parameters in real time and to evaluate their possible evolution in the light of the trend of comparison biometric parameters previously stored in the server 8.

In addition, in the absence of a response and/or notification from the treating physician or the user himself, the Sani app is designed to automatically enable the user/patient to send the request to the managing doctor of the nearest eligible organization (i.e., the organization that is geographically closest to the position of the user terminal 3) and identified by the administrator. Consequently, the organization's manager is provided with the user's data, including, for example, the telephone number associated with the user terminal 3 if the latter is a smartphone, so that the user can possibly be contacted by telephone by the organization after the audio-phonic channel between user and organization has been automatically activated without obtaining a measurable response.

According to one aspect of the present invention, the user terminal 3 is configured to transmit notifications to the medical facility relative to the geographical position of the patient according to the geographical position identified by the geolocalisation devices 5. In other words, the Sani app is designed to communicate the geographical position of the patient to the organization, here the medical facility. In this case, by means of the localization devices 5, the user terminal 3, and therefore the patient, is localised; the geographical position thus detected is transmitted to the external devices 4, possibly together with the identifier so as to inform the organization about the geographical position of the patient at any time subsequent to the first time instant t₀. In this way, the organization is notified of the need to set up the physical and human resources for receiving and the care of the patient. In addition, as also anticipated previously and shown schematically in Figures 3 and 4, the notification of the geographic position of the user terminal 3 (and therefore of the patient) allows the organization, by means of the external devices 4, to determine the time Dt, i.e. the estimated time interval for the patient to reach the organization and/or to provide the patient with the necessary medical assistance and is defined as the time span between the first time instant t₀ and the time instant t₁ (which represents the estimated time instant in which the patient reaches the organization). The time interval required by the organization to set up the measures for the admission and the care of the patient is referred to hereafter as the time Dt' and is defined as the time span between the first time instant t₀ and a time instant t₁', i.e. the time instant in which the setting up of the resources for the management and/or the assistance of the patient is completed. Note that, according to one aspect of the present invention, the times Dt and Dt' are determined by the IT resource 7. In addition, it should be noted that the times Dt and Dt', as will also be evident below, are indicative of a measure of the times taken to provide medical services offered by the organization, as well as their improvement through training and comparison between different organizations; thus, the times Dt and Dt' represent a useful tool for controlling the efficiency of the service of an organization in the management of patients.

Upon notification of the patient's symptoms to the organization and to the patient himself and as previously anticipated, the latter receives a further notification that warns him to go to the organization for the management of the detected alleged pathology; in this phase, according to one aspect of the present invention, the user terminal 3, in particular the Sani app, may provide the patient with a link to a navigation app in order to guide him in reaching the organization. It should be noted that, in one embodiment of the present invention, if the physical conditions and the state of health of the user are severe, the Sani app is designed to automatically send the route to a navigation app, installed on the user terminal 6 or, according to another aspect of the present invention, on a user terminal 3, the latter being close to and for example possessed by another person, in particular by interfacing with the identifier. In this way, the Sani app is designed to issue instructions for taking the patient to the medical facility in situations of extreme need.

It should also be noted that, according to one aspect of the present invention, the Sani app may transmit further notifications to the organization, for example notifications relating to traffic conditions, so as to update in real time the geographical position of the patient and, consequently, the time Dt. In other words, starting from the first time instant t₀ in which the first state of health ("not healthy") is detected, the platform 1 is designed so that the organization is notified of the arrival of a new patient and that the patient is taken to the same organization.

The IT resource 7 is also designed to compare the times Dt and Dt' generated in the previous phase; in particular, when the time Dt' coincides, or overlaps within confidential margins, with the time Dt, it means that the time span needed to set up the resources for the management of the patient is almost equal to or lower than the time span taken by the patient to reach the organization; in other words, the organization, i.e. the medical facility, is able to receive and treat the patient in the first state of health ("not healthy"), previously communicated by notification from the user terminal 3 (in particular from the Sani app). It should be noted that, in one embodiment of the invention, the external devices 4 receive and display notifications adapted to alert the organization of any unforeseen events, route changes or the like that may change the time Dt.

In addition, the IT resource 7 is designed to notify the organization of further information, such as, for example, the patient's route, any critical areas starting from the radius of the distance between the patient and the organization and a possible timeline reporting any delays, impediments and unforeseen events; the IT resource 7 is also designed to allow the organization to contact the patient for any logistical checks and, if necessary, to implement alternative procedures and automatically assign the therapeutic space to another patient.

In other words, the platform 1 allows the flexible and dynamic management of the patients who need to be treated at the organizations involved.

In further embodiments of the present invention, the user terminals 6 can also perform the same operations performed by the IT resource 7; therefore, the user terminals 6 may complement and/or replace the IT resource 7 in terms of operation.

The first time instant t₀ therefore represents the time instant in which supply and demand meet, and the time instant t₁ is the time instant in which the patient reaches the organization, which admits him; it is also assumed that, at the time instant t₁, the organization begins to administer the pre-chosen medical services to the patient. In other words, it is assumed that there are negligible delays between the admission and the treatment of the patient by the medical facility.

The medical services to be administered to the patient in the first state of health ("not healthy") are chosen from an inventory, a predetermined list of medical services and/or protocols appropriate to the case to be treated. According to one aspect of the present invention, the type of health care services to be administered to the patient is also determined according to the classification according to the SEIR model; in other words, depending on the classification category, the health care services to be administered to the patient will vary accordingly, so as to provide *ad hoc* health care services according to the degree of the patient's first state of health.

In addition, during the setting up of the resources and during the same, the IT resource 7 and/or the user terminals 6 can show on the screen for the operators of the organization various information relating to the methods of treatment of the users who come to the organization, for example the number of requests with the relative space-time distances, the times of the procedures of setting up of the resources for the administration of the medical services and the times of execution of the same. In other words, the medical staff can be informed with a certain frequency about the influx of patients at the medical facility. By way of example, for a patient with presumed or confirmed Covid-19 symptoms that are not particularly severe, the medical facility can set up a semi-intensive care unit and administer one or more drugs and, if necessary, provide masks and/or helmets for oxygenation in the event of reduced respiratory capacity. In this way, the organization is able to optimise the management of both the resources available and the times for the management and the treatment of the patient, thus significantly reducing the risk of overloading the organization itself.

At the end of the administration of the healthcare services and having verified that the patient is no longer in the first state of health ("not healthy"), the IT resources 7 and/or the user terminals 6 are designed to assign and transmit to the user terminal 3 a second class, for example according to the SEIR model, relating to a second state of health ("healthy") of the patient at a second time instant t₂, subsequent to the first time instant t₀, as well as to the time instants t₁ and t₁'.

In other words, when the patient reaches a second state of health, defined as "healthy" at time instant t₂ subsequent to the time instants, t₀, t₁ and t₁', the patient is discharged from the organization and removed therefrom; consequently, the user terminal 3 is also removed from the medical facility. In addition, at the time instant t₂, the external devices 4 of the organization update the availability of the resources (e.g. the number of beds and the physical and human resources available), so that they can accept new bookings. In other words, at the time instant t₂, the IT resource 7 and/or the user terminals 6 update the availability of the medical facility following the discharge of a patient now considered healthy. In this step, according to one aspect of the present invention, the IT resource 7 and/or the user terminals 6 update the classification according to the patient's SEIR model and may transmit it to the user terminal 3, which may display it to the patient.

In addition, according to a further aspect of the present invention, if a "not-healthy" state of health falls, for example, within the susceptible category of the SEIR model with biometric parameters or, for example, of the acoustic signals relating to a state of coughing and breathing indicative of an initial form of epidemic or pandemic infection (identified by comparing the data collected by the portable wearable device 2 with emerging and critical reference data stored in the server 8), in order to prevent the patient from exposing himself to the environment of the medical facility (and therefore may generate a chain of contagions), the user terminal 3 sends a notification to the nearby organization so as to arrange for the resources adapted to take the patient to the organization with appropriate vehicles (e.g. ambulance or medical car). In particular, the patient is taken by following a centrifugal, structured route, programmed according to the different zonal requirements, taking into account the geolocalisation data of the patients (determined through the user terminal 3 and the geolocalisation devices 5) by taking the shortest and most efficient route in order to provide the necessary medical service to the patient as soon as possible. In addition, the Sani app is designed to send a notification to the patient in which it can, for example, inform the patient of the arrival times of the vehicles offered by the organization as well as the arrival times to reach the organization, in order to arrange for the medical services he must undergo, such as examinations, blood samples, swabs and therapies. In addition, the sani app is designed to send a notification to the organization about the medical team's departure from the organization (and thus the start of the movement of the vehicles from the organization to the patient's home) as well as from a patient's home (and thus the start of the movement of the vehicles from home to the organization).

It should also be noted that the identifier, the healthcare services provided and further information on the state of health of the patient and the organization's structure can be stored on the server 8; in this way, a remote database relating to the operating conditions of the medical facility is created. It should also be noted that, according to one aspect of the present invention, said server 8 may be shared between different medical facilities, each provided with respective IT resources 7 and/or user terminals 6, in such a way as to create a general database and which allows a more efficient management of the influx of patients in any medical facility. In other words, the platform 1 enables the sharing of information between different medical facilities and different patients being treated and connected via the respective user terminals 3.

Based on what has been previously described, the advantages which the present invention allows to obtain are clear.

In particular, the platform 1 enables the management of activities of daily life through the exchange of identifiers between users (i.e. patients) or between users and organizations, so as to efficiently manage users/patients and the resources available in the organization. In this way, the platform 1 allows to:
- avoid economic collapses;
- improve the quality of life of the world community;
- safeguard the health of the population;
- manage critical health issues such as epidemics, pandemics, ambient disasters and natural disasters in real time;
- identify subjects at risk, thus performing preventive medicine;
- monitor subjects at risk, thus performing telemedicine;
- stabilise subjects at risk, thus performing teletherapy. In particular, teletherapy refers to the release, by means of the portable wearable device 2, in particular by means of special diffusers in the same, and as instructed by the organization or the treating physician, of substances such as nutraceutical and pharmaceutical bioactive nanomolecules, nanomolecules that are hyper-selective for cells or cell sites (also referred to as selective pharmacology). The portable wearable device 2 can also be fitted with multi-frequency laser diode circuits to activate haemoglobin atoms or generate ozone nanoparticles, again with the idea of performing teletherapy;
- admit to dedicated and authorised facilities for the treatment of subjects at risk, e.g. according to the SEIR model, in particular by preparing the resources and administering the medical services necessary for the treatment of the same subjects (personalized medicine);
- recruit appropriate medical and paramedical staff;
- procure medicines and medical devices;
- reduce intervention times; and
- collect data on subjects being treated.

Especially in the event of an epidemic or pandemic, the platform 1 is designed to integrate data from the portable wearable device 2 with ISTAT data, medical bulletins, historical data relative to previous exposures, statistical spread models (such as the SEIR model) and to send notifications to users and organizations

In addition, the interfacing between the portable wearable device 2, the user terminal 3 and the SEIR model allows for a stand-alone network in which different electronic components, such as the LOCs (having local autonomy and adaptive hierarchy) and the IoT systems, collaborate to identify data that can be defined as unmanageable (i.e. data that has caused a threshold or activation level to be exceeded, in a way similar to nodes of a Bayesian network) and activate a series of procedures, effectively carrying out the passage from hardware reality to software reality without the need to have intermediary components that perform conversions from digital to analogue or vice versa. Therefore, the appropriately micro- and/or nanostructured, calibrated and tested LOCs in the execution of their functions detect the data from the outside with the help of the plurality of sensors and return them to the IoT systems in real time; the data received, if considered unmanageable, cause the activation of the procedures described above. Similarly, the sound signals coming from respiratory activity and coughing are processed as smart acoustic interfaces by circuits and algorithms based on machine learning and artificial intelligence methodologies. This therefore results in an efficient exchange of information based on biometric and/or ambient parameters, as well as psychometric parameters, between the medical facility and the patient wearing the portable wearable device 2.

Thus, once a certain amount of data has been stored and processed, the platform 1 is configured as an enabler for the transformation of the autonomous network into a wearable interconnection system in contact with the patient and his biological functions on which to create services of high social value. The information about the state of health, which can be converted into other interpretative models (e.g. according to the SEIR model), is then stored in the system 1 and comprises:
- data related to meta-analytical models (e.g. ISTAT data, previous pandemic experiences and the like);
- data on the organization of the WHO and the NHS; and
- data on geographic and demographic information on the current population and the criticalities thereof referred to mobility, health and the presence of symptoms.

In addition, whenever biometric information (i.e., biometric parameters) and geo-referential information (i.e., the geographical position of the user terminal 3) exceed respective critical values, organized by levels (in particular and for example, individual, household, belonging community, territorial and national system), pre-clinical data emerge; consequently, in an automatic manner, the platform 1 in turn allows generating, in addition to direct information to the users concerned, also coordinated measures starting from the monitoring of the clinical state of the individual, clinical stabilisation, targeted hospitalization.

In addition, the user terminal 3 allows to certify the second state of health ("healthy") of the patient at the social-health and clinical level by:
- monitoring the biometric and/or ambient parameters of the patient through the portable wearable device 2;
- monitoring the psychological state (in particular, the psycho-perceptual sensations);
- monitoring the smart acoustic interfaces;
- monitoring the ambient conditions;
- monitoring the degree of participation in the existential spheres of each patient (e.g. social, family and work sphere according to for example the ICF model, *International Classification of Functioning, Disability and Health);*
- processing a certification of the patient's health and well-being, e.g. by identifying the patient in a classification category of the SEIR model;
- managing the patient at the social-health and preclinical level by means of the identifier;
- updating the identifier according to the change in the psychological and physical conditions, as well as changes in the place and family context of the patient;
- possibility of exchanging the identifier with other users or organizations via wireless connections; and
- possibility of autonomous notification for medical facilities when psychological and physical conditions are detected that need to be treated by a specialist in the medical facility.

In this way, the platform 1 represents a hub for the health of users, including those who are domiciled or reside in remote areas.

The platform 1 therefore enables the efficient management of the rotation of beds, healthcare spaces, staff, supplies, sanitation and maintenance of the medical facility according to demand, waste reduction (spending review) and the efficiency of the health system.

For example, the present portable wearable device may be other than a mask, such as wearable devices of other kind such as earrings, spectacles, bracelets, necklaces, t-shirts, hearing aids, dental prostheses, dental aligners, helmets, cephalic, cervical, cranial-cervical or dorsal-cervical and dorsal-brachial exoskeletons; further, the portable wearable device may comprise a plurality of sensors other than the one disclosed in the present patent application.

In addition, the platform 1 offers various solutions for planning a vaccination campaign based, for example, on the health needs and risk factors identified to ensure the effectiveness of immunisation through the characteristic synchronism of the platform 1 itself.

In particular, the planning (simultaneous or deferred) must take into account at the same time the type of vaccine setting up, its division into doses (single or multiple), storage requirements and its distribution in outpatient facilities (as is currently the case) or at home. According to one aspect of the present invention, the supporting structure 9 provides suitable spaces that can be obtained at the support of the portable wearable device 2 on the soma of the user, for example on the chin (in particular at the chin muscle) or at the dorsal-brachial exoskeleton and the deltoid muscle, as well as adaptable to the format of the commercially available vaccine containers. In addition, the supporting structure 9 offers spaces that can be personalized to allow, by means of micro-diffusers, electromechanical nano-injectors of the MEMS or NEMS type, or by means of electroporation, the release of vaccine doses pre-loaded in the portable wearable device 2 itself at a given instant that allows to maximise the effectiveness of the vaccination campaign. In fact, this condition is possible thanks to the structure of the filter 11, which, according to one embodiment of the present invention described in the preceding paragraphs, has a micro- or nano-architecture for the release of the vaccine form. In addition, the structure of the platform 1 allows users an on-demand and/or automatic pre-screening, the search for the position of the appointment, the geolocalisation of suitable facilities and the route to be taken to reach the suitable facilities or to take the specialist staff to the user.

Vaccines delivered directly to the users in a meta-stable form (e.g. through reversible cryo-crystallisation techniques) allow them to be activated in a pre-programmed timeframe that can be managed by LOC systems through dedicated sensors and IoT services and/or stored in micro-refrigerators so as to allow a synchronous release at a pre-set time and after equalizing the delays due to their distribution. In particular, this pre-set time is defined by means of a software, e.g. of the cloud computing type, which implements an administration program that selects subjects at risk, e.g. starting from the SEIR model, and, on the basis of the values of the biometric parameters, from processing for example acoustic signals or smart acoustic interfaces and on the basis of the psychometric parameters, defines a first level of the state of health in a stable and not worsening phase.

According to one aspect of the present invention, therefore, the time t₀ represents the meeting point between supply and demand, i.e. between the demand for the vaccine and the need to administer it; consequently, the time Dt represents the time required for the user to receive the service or the supply, in parallel with the organization of the supplies (defined by the time Dt') by the medical facility. Furthermore, in this case, the user at the time Dt can be subjected to pre-screening procedures to verify the suitability for vaccination and the priority in vaccination depending on the availability of doses and the time to achieve group immunity; in particular, the need and priority for vaccination is defined starting from the subjects with specific exposure (e.g. health personnel) and from subjects at risk, defined as such for example by the SEIR model, falling within a first level of the state of health. At the same time Dt, the organization administers, according to the protocols shared by the administrator and with the consent of the user candidate for vaccination, a psychometric test to parameterise the mood; if the result of the test is indicative of a not optimal level, the organization transmits, through the audio-phonic channel, reassuring vocal messages with feed-back in response to the measure of the effectiveness of the communication strategy. A time Dt", defined as the time span between the time instants t₂ and t₁, can be used to complete all the procedures indicated in the previous paragraphs, as well as to equalize delays and allow the establishment of a synchronism for the vaccination at time instant t₂, where the platform certifies, e.g. on a blockchain model, that the vaccination has taken place, updates the identifier allowing exchange and verification to regulate the access to facilities or organizations that require it, such as, for example, hospitals, public offices, hotels, airports, gyms, schools or restaurants.

In this way, the platform 1 is designed to allow:
- screening the suitability of the interested users;
- the progressive programming for the vaccinations of the users;
- monitoring the vaccinations and possible side effects;
- monitoring the progress of the immunisation process;
- certification that the user has been vaccinated;
- certification of the type of vaccine; and
- storage of the users who have had that particular vaccine.

Furthermore, the present invention may be related to other application areas, other than the medical-health field, such as the insurance field, relating to professional sports, relating to amateur sports and tourism (for example, relating to travels and tourist excursions).

In addition, the platform may be such that the external devices are electronic devices of facilities other than medical facilities, e.g. schools, gyms, factories, offices and organizations.

In addition, it should be noted that the platform 1 may comprise a larger number of user terminals 3, each provided with respective Sani app, so that more patients are connected to the same medical facility. In addition, the number of IT resources 7 and/or user terminals 6 and/or server 8 may also be greater; by way of example, the platform 1 may comprise a single server 8 and several IT resources 7 and/or user terminals 3, 6, so that several patients and several medical facilities are connected together. In this case, the server 8 acts as a database for several medical facilities and several patients. Alternatively, each medical facility may have respective servers 8 adapted to store data relating solely to that medical facility; in other words, the server 8 is a database that collects information relating to the medical facility and the influx of patients into it.

In addition, according to one embodiment of the present invention, the user terminal 3 is absent and the portable wearable device 2 is designed to interface directly with the external devices 4 and the geolocalisation devices 5; in this case, the portable wearable device 2 may comprise additional electronic components for interfacing with the external devices 4 and the geolocalisation devices 5. Furthermore, in this embodiment, the psychometric data can be evaluated, for example, by means of external electronic devices or by means of the supporting structure 9, such as earphones designed to provide multiple-choice questions and to acquire information relating to the user's psychological state by means of, for example, voice communication with the user himself and be decoded by voice typing and speech recognition algorithms, in order to have the same result obtained using tools such as a keyboard.

In addition, this platform makes it possible to establish a comparative parameterization of the quality system of each of the different medical facilities that are part of the platform 1 for the type of services provided to users by calculating the times Dt' and Dt".

In addition, this platform allows to facilitate the access to any public or private facility, e.g. gyms, bars, shopping malls and medical facilities, without having to carry out evaluation procedures such as temperature taking and personal data release involving pen and paper contact.

In addition, this platform allows monitoring physical effort in competitive team sports correlated to movement strategies on the field.

The platform is also advantageous in that it is built on several IT resources that manage sensitive data; therefore, this platform is suitable for certifying the user's state of health over a certain period of time and the validity of the data collected and relating to the user.

Moreover, this platform is adapted to be treated as a third body that guarantees in a certified and transparent way the date of acquisition of the information and the integrity thereof; de facto, the information coming from the sensors is stored in a database. The retrieval of the information from this database makes it possible to certify that these data, before being provided to private individuals or public bodies, are actually the ones detected by the sensors on the portable wearable device, i.e. that they have not been manipulated and therefore counterfeited in a certain way, as the platform is an automatic and closed environment with regard to the detection and transmission procedures.

Moreover, the traceability process implemented by this platform does not entail any separate costs. In other words, the present invention allows to have a platform that makes it possible to increase the validity of the data and, at the same time, to have an optimisation of the expenses; this advantage makes it possible to guarantee the traceability of the data in a more effective manner, particularly if they are sensitive.

The platform according to the present invention is also based on a blockchain management system and therefore represents a solution to the problem of the validity of sensitive information. The platform could be defined as a sub-family of technologies structured as a chain of blocks containing the data relative to the user and whose validation is entrusted to a consent mechanism, distributed on all the nodes of the network (also defined as Health HUB Platform), i.e. on all the nodes that are authorised to participate in the process of validation of the data to be included in the register. The main characteristics of the blockchain technologies are immutability, transaction traceability and security based on cryptographic techniques; therefore, this platform, based on these characteristics, represents a secure system.

### LIST OF REFERENCE NUMBERS OF THE FIGURES

- 1: system
- 2: portable wearable device
- 3: mobile device
- 4: external devices
- 5: localization devices
- 6: mobile devices
- 7: PC
- 8: server
- 9: supporting structure
- 10: protective shield
- 11: filter
- t₀: first time instant
- t₁: time instant
- t₁': time instant
- Dt: time
- Dt': time
- t₂: second time instant
- Dt": time

## Claims

1. A hardware and/or software platform (1) configured to monitor parameters relative to the psycho-physical conditions of a patient comprising:
- a portable wearable device (2) configured to monitor biometric and/or ambient parameters of a patient and adapted to generate and transmit data indicative of the detected biometric and/or ambient parameters; and
- external devices (4), coupled to the portable wearable device (2),
and wherein the external devices (4) are designed, if the portable wearable device (2) detects a first state of health ("not healthy"), to:
- send, to the patient, a first notification indicating the need to provide medical assistance and/or to take the patient to a medical facility;
- send, to the medical facility, a second notification of the patient's state of health to signal the need for medical assistance and/or hospitalization; and
- determine and store a first time (Dt) between the sending of the first notification and the beginning of the procedures for medical assistance and/or hospitalization by the medical facility and a second time (Dt') between sending of the second notification and the time for activation of the resources necessary for the medical assistance and/or hospitalization of the patient,
wherein the portable wearable device (2) is a mask,
wherein the portable wearable device (2) comprises:
- a supporting structure (9), configured to carry a plurality of sensors configured to detect the biometric and/or ambient parameters; and
- a protective shield (10), carried by the supporting structure (9),
wherein the portable wearable device (2) further comprises a filter (11) carried by the supporting structure (9) and by the protective shield (10) and arranged in a central position with respect to the protective shield (10), the filter (11) being configured to detect in further detail some aspects if required by the state of health and/or the ambient conditions,
and wherein said aspects can be defined through the insertion of nano-structured sensors in the portable wearable device (2) and/or in the filter (11), the nanosensors being designed to sample the air inflow and outflow passing through the respiratory orifices of the patient and to evaluate the quality of the air exhaled and inhaled by the user, said nanosensors, according to the above sampling and evaluation, being alternatively or sequentially configured to:
- activate a wireframe, integrated in the portable wearable device (2) and/or in the filter (11), configured to produce and/or deliver therapeutic ozone; and
- allow the insertion of specific swabs for carrying out rapid anti-viral and/or antibacterial tests in the filter (11) and/or in the portable wearable device (2).

2. The hardware and/or software platform (1) configured to monitor parameters relative to the psycho-physical conditions of a patient according to claim 1, further comprising a user terminal (3) coupled with the portable wearable device (2) and configured to receive the data relative to the biometric and/or ambient parameters of the patient detected by the portable wearable device (2), said user terminal (3) being configured to generate data relative to psychometric parameters relating to the psychological conditions of the patient and process the data relative to the detected biometric and/or ambient parameters and to the psychometric parameters in order to generate an identifier containing information relative to the overall state of psycho-physical health of the patient,
wherein the external devices (4) are coupled to the user terminal (3) and are configured to receive and process the identifier,
and wherein the external devices (4) are designed to send to the patient, through the user terminal (3), the first notification indicating the need for medical assistance and/or the need to take the patient to a medical facility if the identifier indicates the first state of health.

3. The hardware and/or software platform (1) configured to monitor parameters relative to the psycho-physical conditions of a patient according to claim 1 and furthermore designed to allow the insertion of devices for the programmed administration of vaccines in the filter (11) and/or in the portable wearable device (2).

4. The hardware and/or software platform (1) configured to monitor parameters relative to the psycho-physical conditions of a patient according to claim 1, wherein the filter (11) is configured to carry out a feedback and/or feed-forward correction of the air quality, modifying the microclimate in such a way that the portable wearable device (2) allows for the creation of a climate or a microclimate the conditions of which are favorable to the patient's health and unfavorable to the survival, residence and proliferation of viruses.

5. The hardware and/or software platform (1) configured to monitor parameters relative to the psycho-physical conditions of a patient according to claim 1 or 4, wherein the filter (11) is furthermore designed to provide a "nano-architecture" specular to the shape, dimensions and spatial representation of a particular virus, thus being able to "trap" it.

6. The hardware and/or software platform (1) configured to monitor parameters relative to the psycho-physical conditions of a patient according to any one of claims 1, 4-5, wherein the filter (11) is of a removable type, namely it can be removed, for example, to be analysed in structural or microbiological terms in order to find a known virus and/or a variation thereof, and replaced.

7. The hardware and/or software platform (1) configured to monitor parameters relative to the psycho-physical conditions of a patient according to any one of the preceding claims, wherein:
- biometric parameters are indicative of the state of health of the patient and comprise at least one of body temperature, oxygen (O₂) saturation, CO₂ concentration, cardiac frequency, voice analysis, cough intensity and quality analysis; and
- ambient parameters are indicative of the ambient conditions surrounding the patient and comprise at least one of quantity of CO₂ and fine particulate in the air.

8. The hardware and/or software platform (1) configured to monitor parameters relative to the psycho-physical conditions of a patient according to any one of the preceding claims, wherein the external devices (4) comprise:
- user terminals (6) configured to send requests for sharing of the identifier generated by the user terminal (3) and receive said identifier upon acceptance of the sharing by the user terminal (3) as instructed by the patient;
- hardware and/or software IT resources (7) configured to receive and/or store the identifier coming from the user terminal (3); and
- servers (8) configured to receive the identifier from the user terminal (3) and/or data relative to the medical facility and to the state of health of the patient during administration of the medical services.

9. The hardware and/or software platform (1) configured to monitor parameters relative to the psycho-physical conditions of a patient according to claim 8, wherein the user terminal (3) is designed to assign a first class relative to the first state of health ("not healthy") of the patient according to a model in a first time instant (t₀), and wherein the IT resources (7) and/or the user terminals (6) are designed to assign and transmit, to the user terminal (3), a second class relative to a second state of health ("healthy") of the patient in a second time instant (t₂), subsequent to the first time instant.

10. The hardware and/or software platform (1) configured to monitor parameters relative to the psycho-physical conditions of a patient according to any one of the preceding claims, further comprising geolocalisation devices (5) configured to:
- identify the geographical position of the user terminal (3) of the patient; and
- generate routes, displayable on the user terminal (3), for taking the patient to the medical facility,
wherein the user terminal (3) is configured to inform the patient of routes followed by a medical team to reach the patient's domicile in the event of home medical assistance,
and wherein the user terminal (3) is configured to transmit notifications to the medical facility relative to the geographical position of the patient according to the geographical position identified by the geolocalisation devices (5).

## Patentansprüche

1. Hardware- und/oder Software-Plattform (1), die zur Überwachung von Parametern bezüglich des psychophysischen Zustands eines Patienten konfiguriert ist, umfassend:
- eine tragbare, am Körper tragbare Vorrichtung (2),die zur Überwachung von Parametern bezüglich des psychophysischen Zustands eines Patienten konfiguriert ist und die so angepasst ist, dass sie Daten erzeugt und überträgt, die die erfassten biometrischen Parameter und/oder Umgebungsparameter anzeigen; und
- externe Vorrichtungen (4), die mit der tragbaren, am Körper tragbaren Vorrichtung (2) verbunden sind,
und wobei die externen Vorrichtungen (4) so ausgelegt sind, dass sie, wenn die tragbare, am Körper tragbare Vorrichtung (2) einen ersten Gesundheitszustand ("nicht gesund") erfasst, dazu dienen:
- eine erste Benachrichtigung an den Patienten zu senden, in der auf die Notwendigkeit hingewiesen wird, medizinische Hilfe bereitzustellen und/oder den Patienten in eine medizinische Einrichtung zu bringen;
- eine zweite Benachrichtigung über den Gesundheitszustand des Patienten an die medizinische Einrichtung zu senden, um die Notwendigkeit medizinischer Hilfe und/oder eines Krankenhausaufenthalts zu signalisieren; und
- eine erste Zeit (Dt) zwischen dem Senden der ersten Benachrichtigung und dem Beginn der Prozeduren für medizinische Hilfe und/oder Krankenhausaufenthalt durch die medizinische Einrichtung und eine zweite Zeit (Dt') zwischen dem Senden der zweiten Benachrichtigung und dem Zeitpunkt für die Aktivierung der für die medizinische Hilfe und/oder den Krankenhausaufenthalt des Patienten erforderlichen Ressourcen zu bestimmen und zu speichern,
wobei die tragbare, am Körper tragbare Vorrichtung (2) eine Maske ist,
wobei die tragbare, am Körper tragbare Vorrichtung (2) umfasst:
- eine Trägerstruktur (9), die so konfiguriert ist, dass sie eine Vielzahl von Sensoren trägt, die so konfiguriert sind, dass sie die biometrischen Parameter und/oder Umgebungsparameter erfassen; und
- ein Schutzschild (10), das von der Trägerstruktur (9) getragen wird,
wobei die tragbare, am Körper tragbare Vorrichtung (2) ferner einen Filter (11) umfasst, der von der Trägerstruktur (9) und von dem Schutzschild (10) getragen wird und in einer zentralen Position in Bezug auf das Schutzschild (10) angeordnet ist, wobei der Filter (11) so konfiguriert ist, dass er einige Aspekte im Detail erfasst, wenn dies aufgrund des Gesundheitszustands und/oder der Umgebungsbedingungen erforderlich ist,
und wobei die genannten Aspekte durch das Einsetzen von nanostrukturierten Sensoren in die tragbare, am Körper tragbare Vorrichtung (2) und/oder in den Filter (11) definiert werden können, wobei die Nanosensoren so ausgelegt sind, dass sie die durch die Atemöffnungen des Patienten ein- und ausströmende Luft abtasten und die Qualität der vom Benutzer ausgeatmeten und eingeatmeten Luft bewerten, wobei die Nanosensoren gemäß der oben genannten Abtastung und Bewertung alternativ oder nacheinander so konfiguriert sind zum:
- Aktivieren eines in die tragbare, am Körper tragbare Vorrichtung (2) und/oder in den Filter (11) integrierten Drahtgestells, das so konfiguriert ist, dass es therapeutisches Ozon erzeugt und/oder abgibt; und
- Ermöglichen des Einführens spezifischer Tupfer zur Durchführung von antiviralen und/oder antibakteriellen Schnelltests in den Filter (11) und/oder in die tragbare, am Körper tragbare Vorrichtung (2).

2. Hardware- und/oder Softwareplattform (1), die zur Überwachung von Parametern bezüglich des psychophysischen Zustands eines Patienten nach Anspruch 1 konfiguriert ist, und die ferner ein Benutzerterminal (3) umfasst, das mit der tragbaren, am Körper tragbaren Vorrichtung (2) gekoppelt und so konfiguriert ist, dass es die Daten in Bezug auf die biometrischen Parameter und/oder Umgebungsparameter des Patienten empfängt, die von der tragbaren, am Körper tragbaren Vorrichtung (2) erfasst werden, wobei das Benutzerterminal (3) so konfiguriert ist, dass es Daten in Bezug auf psychometrische Parameter erzeugt, die sich auf den psychologischen Zustand des Patienten beziehen, und die Daten in Bezug auf die erfassten biometrischen Parameter und/oder Umgebungsparameter und die psychometrischen Parameter verarbeitet, um einen Benutzernamen zu erzeugen, der Informationen in Bezug auf den Gesamtzustand der psycho-physischen Gesundheit des Patienten enthält,
wobei die externen Vorrichtungen (4) mit dem Benutzerterminal (3) gekoppelt und so konfiguriert sind, dass sie den Benutzernamen empfangen und verarbeiten,
und wobei die externen Vorrichtungen (4) so ausgelegt sind, dass sie über das Benutzerterminal (3) die erste Benachrichtigung an den Patienten senden, die auf die Notwendigkeit medizinischer Hilfe und/oder die Notwendigkeit hinweist, den Patienten in eine medizinische Einrichtung zu bringen, wenn der Benutzername den ersten Gesundheitszustand anzeigt.

3. Hardware- und/oder Softwareplattform (1), die zur Überwachung von Parametern bezüglich des psychophysischen Zustands eines Patienten nach Anspruch 1 konfiguriert ist, und die darüber hinaus so ausgelegt ist, dass sie das Einsetzen von Vorrichtungen für die programmierte Verabreichung von Impfstoffen in den Filter (11) und/oder in die tragbare, am Körper tragbare Vorrichtung (2) ermöglicht.

4. Hardware- und/oder Softwareplattform (1), die zur Überwachung von Parametern bezüglich des psychophysischen Zustands eines Patienten nach Anspruch 1 konfiguriert ist, wobei der Filter (11) so konfiguriert ist, dass er eine Rückkopplungs- und/oder Vorwärtskorrektur der Luftqualität durchführt, die das Mikroklima so verändert, dass die tragbare, am Körper tragbare Vorrichtung (2) die Schaffung eines Klimas oder eines Mikroklimas ermöglicht, dessen Bedingungen für die Gesundheit des Patienten günstig und für das Überleben, das Verbleiben und die Vermehrung von Viren ungünstig sind.

5. Hardware- und/oder Software-Plattform (1), die zur Überwachung von Parametern bezüglich des psychophysischen Zustands eines Patienten nach Anspruch 1 oder 4 konfiguriert ist, wobei der Filter (11) außerdem so ausgelegt ist, dass er eine "Nano-Architektur" bereitstellt, die der Form, den Abmessungen und der räumlichen Darstellung eines bestimmten Virus entspricht, und somit in der Lage ist, diesen "einzufangen".

6. Hardware- und/oder Software-Plattform (1), die zur Überwachung von Parametern bezüglich des psychophysischen Zustands eines Patienten nach Anspruch 1, 4 bis 5 konfiguriert ist, wobei der Filter (11) von einem abnehmbaren Typ ist, d. h. beispielsweise entfernt werden kann, um strukturell oder mikrobiologisch analysiert zu werden, um ein bekanntes Virus und/oder eine Variation davon zu finden, und ersetzt werden kann.

7. Hardware- und/oder Software-Plattform (1), die zur Überwachung von Parametern bezüglich des psychophysischen Zustands eines Patienten nach einem der vorstehenden Ansprüche konfiguriert ist, wobei:
- biometrische Parameter den Gesundheitszustand des Patienten anzeigen und mindestens eines der Folgenden umfassen: Körpertemperatur, Sauerstoff(O₂)-Sättigung, CO₂-Konzentration, Herzfrequenz, Stimmanalyse, Hustenintensitäts- und qualitätsanalyse; und
- die Umgebungsparameter ein Indikator für die Umgebungsbedingungen rund um den Patienten sind und mindestens eines CO₂- und Feinstaubmenge in der Luft umfassen.

8. Hardware- und/oder Softwareplattform (1), die zur Überwachung von Parametern bezüglich des psychophysischen Zustands eines Patienten nach einem der vorstehenden Ansprüche konfiguriert ist, wobei die externen Geräte (4) umfassen:
- Benutzerterminals (6), die so konfiguriert sind, dass sie Anfragen zur Freigabe des vom Benutzerterminal (3) erzeugten Benutzernamen senden und den Benutzernamen nach Annahme der Freigabe durch das Benutzerterminal (3) gemäß den Anweisungen des Patienten empfangen;
- Hardware- und/oder Software-IT-Ressourcen (7), die so konfiguriert sind, dass sie den vom Benutzerterminal (3) kommende Benutzernamen empfangen und/oder speichern; und
- Server (8), die so konfiguriert sind, dass er den Benutzernamen vom Benutzerterminal (3) und/oder Daten in Bezug auf die medizinische Einrichtung und den Gesundheitszustand des Patienten während der Verabreichung der medizinischen Dienstleistungen empfangen.

9. Hardware- und/oder Softwareplattform (1), die zur Überwachung von Parametern bezüglich des psychophysischen Zustands eines Patienten nach Anspruch 8 konfiguriert ist, wobei das Benutzerendgerät (3) dazu ausgelegt ist, in einem ersten Zeitpunkt (to) eine erste Klasse bezüglich des ersten Gesundheitszustands ("nicht gesund") des Patienten gemäß einem Modell zuzuordnen, und wobei die IT-Ressourcen (7) und/oder die Benutzerterminals (6) dazu ausgelegt sind, dem Benutzerterminal (3) zu einem zweiten Zeitpunkt (t₂), der auf den ersten Zeitpunkt folgt, eine zweite Klasse bezüglich eines zweiten Gesundheitszustands ("gesund") des Patienten zuzuordnen und zu übertragen.

10. Hardware- und/oder Software-Plattform (1), die zur Überwachung von Parametern bezüglich des psychophysischen Zustands eines Patienten nach einem der vorstehenden Ansprüche konfiguriert ist, ferner umfassend Geolokalisierungsvorrichtungen (5), die so konfiguriert sind zum:
- Identifizieren der geografischen Position des Benutzerterminals (3) des Patienten; und
- Erstellen von auf dem Benutzerterminal (3) anzeigbaren Routen für den Transport des Patienten zur medizinischen Einrichtung,
wobei das Benutzerterminal (3) so konfiguriert ist, dass es den Patienten über die Routen informiert, die ein medizinisches Team im Falle einer häuslichen medizinischen Hilfe zum Wohnsitz des Patienten führt,
und wobei das Benutzerterminal (3) so konfiguriert ist, dass es Benachrichtigungen an die medizinische Einrichtung bezüglich der geografischen Position des Patienten gemäß der von den Geolokalisierungsvorrichtungen (5) identifizierten geografischen Position übermittelt.

## Revendications

1. Plate-forme matérielle et/ou logicielle (1) configurée pour surveiller des paramètres relatifs aux conditions psychophysiques d'un patient, comprenant :
- un dispositif portable à porter sur soi (2) configuré pour surveiller des paramètres biométriques et/ou ambiants d'un patient et adapté pour générer et transmettre des données indicatives des paramètres biométriques et/ou ambiants détectés ; et
- des dispositifs externes (4), couplés au dispositif portable à porter sur soi (2),
et dans laquelle les dispositifs externes (4) sont conçus, si le dispositif portable à porter sur soi (2) détecte un premier état de santé (« pas en bonne santé »), pour :
- envoyer, au patient, une première notification indiquant la nécessité de fournir une assistance médicale et/ou d'emmener le patient dans un établissement médical ;
- envoyer, à l'établissement médical, une seconde notification de l'état de santé du patient pour signaler la nécessité d'une assistance médicale et/ou d'une hospitalisation ; et
- déterminer et stocker un premier temps (Dt) entre l'envoi de la première notification et le début des procédures d'assistance médicale et/ou d'hospitalisation par l'établissement médical et un second temps (Dt') entre l'envoi de la seconde notification et le temps de l'activation des ressources nécessaires pour l'assistance médicale et/ou l'hospitalisation du patient,
dans laquelle le dispositif portable à porter sur soi (2) est un masque,
dans laquelle le dispositif portable à porter sur soi (2) comprend :
- une structure de support (9), configurée pour porter une pluralité de capteurs configurés pour détecter les paramètres biométriques et/ou ambiants ; et
- un écran de protection (10), porté par la structure de support (9),
dans laquelle le dispositif portable à porter sur soi (2) comprend en outre un filtre (11) porté par la structure de support (9) et par l'écran de protection (10) et agencé à une position centrale par rapport à l'écran de protection (10), le filtre (11) étant configuré pour détecter plus en détail certains aspects si cela est requis par l'état de santé et/ou les conditions ambiantes,
et dans laquelle lesdits aspects peuvent être définis par le biais de l'insertion de capteurs nanostructurés dans le dispositif portable à porter sur soi (2) et/ou dans le filtre (11), les nanocapteurs étant conçus pour échantillonner le flux entrant et le flux sortant d'air passant par les orifices respiratoires du patient et pour évaluer la qualité de l'air expiré et inhalé par l'utilisateur, lesdits nanocapteurs, selon l'échantillonnage et l'évaluation susmentionnés, étant alternativement ou en séquence configurés pour :
- activer une armature filaire, intégrée dans le dispositif portable à porter sur soi (2) et/ou dans le filtre (11), configurée pour produire et/ou distribuer de l'ozone thérapeutique ; et
- permettre l'insertion d'écouvillons spécifiques pour effectuer des tests antiviraux et/ou antibactériens rapides dans le filtre (11) et/ou dans le dispositif portable à porter sur soi (2).

2. Plate-forme matérielle et/ou logicielle (1) configurée pour surveiller des paramètres relatifs aux conditions psychophysiques d'un patient selon la revendication 1, comprenant en outre un terminal d'utilisateur (3) couplé au dispositif portable à porter sur soi (2) et configuré pour recevoir les données relatives aux paramètres biométriques et/ou ambiants du patient détectés par le dispositif portable à porter sur soi (2), ledit terminal d'utilisateur (3) étant configuré pour générer des données relatives à des paramètres psychométriques relatifs aux conditions psychologiques du patient et traiter les données relatives aux paramètres biométriques et/ou ambiants détectés et aux paramètres psychométriques afin de générer un identifiant contenant des informations relatives à l'état de santé psychophysique global du patient,
dans laquelle les dispositifs externes (4) sont couplés au terminal d'utilisateur (3) et sont configurés pour recevoir et traiter l'identifiant,
et dans laquelle les dispositifs externes (4) sont conçus pour envoyer au patient, par le biais du terminal d'utilisateur (3), la première notification indiquant la nécessité d'une assistance médicale et/ou la nécessité d'emmener le patient dans un établissement médical si l'identifiant indique le premier état de santé.

3. Plate-forme matérielle et/ou logicielle (1) configurée pour surveiller des paramètres relatifs aux conditions psychophysiques d'un patient selon la revendication 1 et conçue en outre pour permettre l'insertion de dispositifs pour l'administration programmée de vaccins dans le filtre (11) et/ou dans le dispositif portable à porter sur soi (2).

4. Plate-forme matérielle et/ou logicielle (1) configurée pour surveiller des paramètres relatifs aux conditions psychophysiques d'un patient selon la revendication 1, dans laquelle le filtre (11) est configuré pour effectuer une correction à rétroaction et/ou à action directe de la qualité de l'air, en modifiant le microclimat d'une manière telle que le dispositif portable à porter sur soi (2) permette la création d'un climat ou d'un microclimat dont les conditions sont favorables à la santé du patient et défavorables à la survie, à la résidence et à la prolifération des virus.

5. Plate-forme matérielle et/ou logicielle (1) configurée pour surveiller des paramètres relatifs aux conditions psychophysiques d'un patient selon la revendication 1 ou 4, dans laquelle le filtre (11) est en outre conçu pour fournir une « nano-architecture » spéculaire à la forme, aux dimensions et à la représentation spatiale d'un virus particulier, étant ainsi apte à le « piéger ».

6. Plate-forme matérielle et/ou logicielle (1) configurée pour surveiller des paramètres relatifs aux conditions psychophysiques d'un patient selon l'une quelconque des revendications 1, 4 et 5, dans laquelle le filtre (11) est d'un type amovible, autrement dit il peut être retiré, par exemple, pour être analysé en termes structurels ou microbiologiques afin de trouver un virus connu et/ou une variation de celui-ci, et remplacé.

7. Plate-forme matérielle et/ou logicielle (1) configurée pour surveiller des paramètres relatifs aux conditions psychophysiques d'un patient selon l'une quelconque des revendications précédentes, dans laquelle :
- les paramètres biométriques sont indicatifs de l'état de santé du patient et comprennent au moins l'une parmi la température corporelle, la saturation en oxygène (O₂), la concentration en CO₂, la fréquence cardiaque, une analyse de la voix, l'intensité de la toux et une analyse de qualité ; et
- les paramètres ambiants sont indicatifs des conditions ambiantes environnant le patient et comprennent au moins l'une parmi la quantité de CO₂ et de particules fines dans l'air.

8. Plate-forme matérielle et/ou logicielle (1) configurée pour surveiller des paramètres relatifs aux conditions psychophysiques d'un patient selon l'une quelconque des revendications précédentes, dans laquelle les dispositifs externes (4) comprennent :
- des terminaux d'utilisateur (6) configurés pour envoyer des requêtes de partage de l'identifiant généré par le terminal d'utilisateur (3) et recevoir ledit identifiant lors de l'acceptation du partage par le terminal d'utilisateur (3) conformément aux instructions du patient ;
- des ressources informatiques matérielles et/ou logicielles (7) configurées pour recevoir et/ou stocker l'identifiant provenant du terminal d'utilisateur (3) ; et
- des serveurs (8) configurés pour recevoir l'identifiant provenant du terminal d'utilisateur (3) et/ou des données relatives à l'établissement médical et à l'état de santé du patient pendant l'administration des services médicaux.

9. Plate-forme matérielle et/ou logicielle (1) configurée pour surveiller des paramètres relatifs aux conditions psychophysiques d'un patient selon la revendication 8, dans laquelle le terminal d'utilisateur (3) est conçu pour attribuer une première classe relative au premier état de santé (« pas en bonne santé ») du patient selon un modèle à un premier instant temporel (t₀), et dans laquelle les ressources informatiques (7) et/ou les terminaux d'utilisateur (6) sont conçus pour attribuer et transmettre, au terminal d'utilisateur (3), une seconde classe relative à un second état de santé (« en bonne santé ») du patient à un second instant (t₂), postérieur au premier instant.

10. Plate-forme matérielle et/ou logicielle (1) configurée pour surveiller des paramètres relatifs aux conditions psychophysiques d'un patient selon l'une quelconque des revendications précédentes, comprenant en outre des dispositifs de géolocalisation (5) configurés pour :
- identifier la position géographique du terminal d'utilisateur (3) du patient ; et
- générer des itinéraires, aptes à être affichés sur le terminal d'utilisateur (3), pour emmener le patient dans l'établissement médical,
dans laquelle le terminal d'utilisateur (3) est configuré pour informer le patient d'itinéraires suivis par une équipe médicale pour atteindre le domicile du patient dans le cas d'une assistance médicale à domicile,
et dans laquelle le terminal d'utilisateur (3) est configuré pour transmettre des notifications à l'établissement médical relatives à la position géographique du patient selon la position géographique identifiée par les dispositifs de géolocalisation (5).
